(19) Europäisches Patentamt
European Patent Office
Office européen des brevets

(11) Veröffentlichungsnummer: **0 315 913 B1**

# (12) EUROPÄISCHE PATENTSCHRIFT

(45) Veröffentlichungstag der Patentschrift: **10.06.92**

(21) Anmeldenummer: **88118409.7**

(22) Anmeldetag: **04.11.88**

(51) Int. Cl.5: **A61K 7/06**, A61K 7/48, A61K 31/085, A61K 31/19, A61K 31/235, A61K 31/22

## (54) Sebosuppressive topische Zubereitungen.

(30) Priorität: **12.11.87 DE 3738406**

(43) Veröffentlichungstag der Anmeldung:
**17.05.89 Patentblatt 89/20**

(45) Bekanntmachung des Hinweises auf die Patenterteilung:
**10.06.92 Patentblatt 92/24**

(84) Benannte Vertragsstaaten:
**AT BE CH DE ES FR GB GR IT LI LU NL SE**

(56) Entgegenhaltungen:
**EP-A- 0 114 051**
**FR-A- 2 528 826**

(73) Patentinhaber: **Henkel Kommanditgesellschaft auf Aktien**
**Postfach 1100 Henkelstrasse 67**
**W-4000 Düsseldorf 1(DE)**

(72) Erfinder: **Möller, Hinrich, Dr.**
**Haydnstrasse 27**
**W-4019 Monheim(DE)**
Erfinder: **Banduhn, Norbert, Dr.**
**Gerhard-Hauptmann-Strasse 44**
**W-4006 Erkrath 1(DE)**

EP 0 315 913 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von Alkyl- und Alkenyl-Arylether-Derivaten spezieller Struktur als antiseborrhoische Wirkstoffe zur Herstellung von topischen, pharmazeutischen oder kosmetischen Mitteln mit sebosuppressiver Wirkung.

Übermäßige Absonderungen der Talgdrüsen der Oberhaut können zu krankhaften Hautzuständen führen. In häufiger vorkommenden, leichteren Fällen stellen sie ein kosmetisches Problem dar, das sich durch fettiges Aussehen der Haare oder ein glänzendes, öliges Aussehen der Haut manifestiert. Die moderne Kosmetik ist daher bemüht, durch geeignete topisch anwendbare Zubereitungen die Sekretion der Talgdrüsen zu normalisieren und dem Haar und der Haut wieder ein ansprechendes Aussehen zu verleihen.

4-Alkoxy-benzylalkohole und -phenetole sind bereits in der deutschen Patentanmeldung DE-A 33 32 505, 4-Alkoxy-benzoesäureester in den deutschen Patentanmeldungen DE-A 31 21 064, DE-A 35 00 971 und der europäischen Patentanmeldung EP-A 114 051, 4-Alkoxybenzoesäuren und deren Salze in den deutschen Patentanmeldungen DE-A 30 47 106 und DE-A 35 00 972, Alkoxyzimtsäureester in der deutschen Patentanmeldung DE-A 31 21 091 und $C_1$-$C_6$-Alkoxyphenylpropionsäureester in der deutschen Patentanmeldung DE-A 30 47 106 als antiseborrhoische Zusätze für kosmetische Zubereitungen vorgeschlagen worden.

Obwohl die in den genannten Druckschriften vorgeschlagenen Verbindungen eine deutliche antiseborrhoische Wirkung zeigen, besteht doch weiterhin ein Bedürfnis an Zubereitungen mit einer erhöhten Wirksamkeit bei niedrigen Anwendungskonzentrationen. Es wurden nunmehr Verbindungen gefunden, die bereits bei Anwendungskonzentrationen von 0,001 Gew.-% und darunter in für topische Applikation geeigneten Trägern eine gute antiseborrhoische Wirkung zeigen.

Gegenstand der Erfindung ist daher die Verwendung von Alkyl-und Alkenyl-Arylether-Derivaten der Formel (I)

(I) 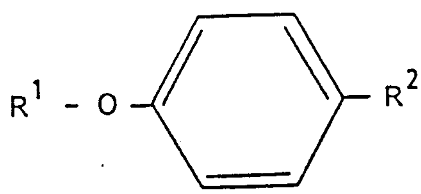

in der $R^1$ eine gesättigte, cycloaliphatische Alkylgruppe mit 6 bis 20 C-Atomen oder eine einfach oder mehrfach ungesättigte, lineare, verzweigte oder cycloaliphatische Alkylgruppe mit 6 bis 20 C-Atomen und $R^2$ eine Hydroxyalkylgruppe mit 1 bis 4 C-Atomen, eine Hydroxyalkenylgruppe mit 3 oder 4 C-Atomen, eine Gruppe $-COOR^3$, $-CH_2-COOR^3$, $-CH_2-CH_2-COOR^3$ oder $-CH=CH-COOR^3$ ist, worin $R^3$ eine Alkylgruppe mit 1 bis 4 C-Atomen, eine Hydroxyalkylgruppe mit 2 bis 4 C-Atomen, eine Alkoxyalkylgruppe mit 1 bis 4 C-Atomen in der Alkoxy- und 2 bis 4 C-Atomen in der Alkylgruppe oder Wasserstoff oder ein salzbildendes Kation ist, als antiseborrhoische Wirkstoffe zur Herstellung von topischen, pharmazeutischen und kosmetischen Mitteln mit sebosuppressiver Wirkung.

In den Verbindungen der Formel (I) kann $R^1$ z. B. eine cycloaliphatische Alkylgruppe sein, d. h. eine Alkylgruppe, die einen Cyclopentan-, Cyclohexan- oder Cycloheptanring enthält, wobei diese Cycloalkanringe mit einer oder mehreren niederen Alkylgruppen substituiert sein können. Insgesamt soll die cycloaliphatische Alkylgruppe etwa 6 bis 20 C-Atome enthalten. So kann $R^1$ z. B. eine Cyclohexylmethyl-, eine 1- oder 2-Cyclohexylethyl-, eine 1-, 2- oder 3-Cyclohexyl-propyl-, eine 1-, 2-, 3-oder 4-Cyclohexyl-butyl-, eine 4-Cylohexyl-2-butyl-, eine 3- oder 5-Cyclohexyl-pentyl-, eine 6-Cyclohexyl-hexyl-, eine 4-(1,1,3-Trimethyl-2-cyclohexyl)-2-butyl-, eine 4-(3,3,5-Trimethyl-cyclohexyl)-2-butyl-, eine 4-(3,3,5-Trimethyl-cyclohexyl)-butyl- oder eine 3-(3,3,5-Trimethyl-cyclohexyl)-propyl-gruppe sein.

$R^1$ kann auch eine einfach oder mehrfach ungesättigte, cycloaliphatische Gruppe mit 6 bis 20 C-Atomen sein, z. B. eine 4-Cyclohexyl-but-3-en-2-yl-, eine 4-(1,1,3-Trimethyl-2-cyclohexyl)-but-3-en-2-yl-, eine 4-(1,1,3-Trimethyl-cyclohex-1-en-2-yl)-but-3-en-2-yl-,eine 4-Trimethyl-cyclohex-1-en-2-yl)-2-butyl-, eine 4-(1,1,3-Trimethyl-cyclohex-3-en-2-yl)-2-butyl- oder eine 4-(3,3,5-Trimethyl-1(6)-cyclohexenyl-2-butyl-gruppe.

Bevorzugt ist $R^1$ eine Cyclohexyl-alkyl- oder eine Mono-, Di-oder Trimethyl-cyclohexyl-alkylgruppe mit insgesamt 6 bis 20 C-Atomen.

Schließlich kann $R^1$ auch eine einfach oder mehrfach ungesättigte, lineare oder verzweigte aliphatische Alkylgruppe mit 6 bis 20 C-Atomen sein. Gruppen dieser Art sind z. B. die 11-Undecenyl-, die Oleyl-, die

Linoleyl- die Elaidyl-, die Linolaidyl-, die Arachidyl- und die Erucyl-gruppe. Bevorzugte, ungesättigte Alkenylgruppen sind die verzweigten, ungesättigten, von Terpenalkoholen abgeleiteten Gruppen wie z. B. die Geranyl-, die Neryl-, die Nerolidyl-, die Farnesyl-, die cis- und trans-6,10-Dimethyl-5,9-undecadienyl-, die beta-Citronellyl-, die Phytyl- und die Isophytyl-gruppe.

Wenn $R^2$ eine Estergruppe ist, so ist der Alkoholrest $R^3$ z. B. ein Methyl-, Ethyl-, Propyl-, Isopropyl-, Butyl-, 2-Butyl-, 2-Hydroxylethyl-, 2- oder 3-Hydroxypropyl-, 2-Methoxyethyl-, 2-oder 3-Methoxypropylrest.

$R^2$ ist bevorzugt eine Gruppe $-CH_2OH$, $-CH=CH-CH_2OH$, $-COOR^3$, $-CH_2-CH_2-COOR^3$ oder $-CH=CH-COOR^3$, worin $R^3$ bevorzugt Wasserstoff, ein salzbildendes Kation oder eine Alkylgruppe mit 1 bis 4 C-Atomen ist.

Die Alkyl- und Alkenyl-Aryletherderivate der allgemeinen Formel (I) sind teilweise neu, als Verbindungen des aus den obengenannten Druckschriften bekannten Grundtyps lassen sie sich auch nach den dort angegebenen, literaturbekannten allgemeinen Syntheseverfahren herstellen:
Die p-Alkoxybenzoesäure-methylester werden z. B. durch Alkylierung von p-Hydroxybenzoesäure-methylester mit den Halogeniden der Formel $R^1$-X (worin X z. B. Chlor oder Brom ist) oder mit entsprechenden Sulfaten oder Sulfonsäureestern hergestellt.

Die Hydroxyalkyl- und Alkoxyalkylester können dann aus den entsprechenden p-Alkoxy-benzoesäuremethylestern durch Umesterung mit den jeweiligen Alkoholkomponenten $R^3$-OH in Gegenwart alkalischer Katalysatoren, wie z. B. Natriumalkoholaten, hergestellt werden.

Man kann auch umgekehrt zunächst die Veresterung der p-Hydroxybenzoesäure und danach die Alkylierung durchführen.

Analog zu den Alkoxybenzoesäure-Estern lassen sich auch die Alkoxy-zimtsäureester, die p-Alkoxyphenyl-propionsäureester und die p-Alkoxy-phenylethansäureester aus den entsprechenden p-Hydroxyphenyl-carbonsäuremethylestern herstellen.

Die freien Säuren, in welchen $R^3$ = Wasserstoff ist, lassen sich aus den entsprechenden Methylestern leicht durch Verseifung (Hydrolyse) gewinnen. Durch Neutralisation mit Basen lassen sie sich in die Salze überführen, in welchen $R^3$ das salzbildende Kation ist. Besonders gut geeignete, dermatologisch verträgliche Salze sind vor allem die Alkali- und Erdalkalisalze, z. B. die Natrium-, Kalium-, Calcium- oder Magnesiumsalze, aber auch die Ammonium- und Alkanolammoniumsalze, z. B. das Monoethanolammoniumsalz, das Isopropanolammoniumsalz oder das Triethanolammoniumsalz. Es sind aber auch Salze anderer Basen wirksam, soweit diese ausreichend dermatologisch verträglich sind.

Die Verbindungen der allgemeinen Formel (I), in welchen $R^2$ eine Hydroxyalkylgruppe ist, lassen sich nach literaturbekannten Verfahren aus den entsprechenden Carbonsäuremethylestern durch Reduktion mit komplexen Metallhydriden, z. B. mit Natriumborhydrid, Lithium-aluminiumhydrid oder mit Natrium-bis-(2-methoxyethoxy)-aluminiumhydrid (Vitride®) herstellen. Beispiele für die Herstellung von nicht literaturbekannten Alkyl- oder Alkenyl-Arylether-Derivaten der Formel (I) werden im Beispielteil angegeben.

Die Alkyl- und Alkenyl-Arylether-Derivate der Formel (I) besitzen eine ausgeprägte sebosuppressive Wirkung, wobei viele der Produkte bereits bei sehr niedrigen Anwendungskonzentrationen eine merkliche sebostatische und antiseborrhoische Wirkung auf der Haut entfalten. Sie besitzen darüber hinaus eine ausgezeichnete Haut- und Schleimhautverträglichkeit. Die Alkyl- und Alkenyl-Arylether-Derivate der Formel (I) werden bevorzugt in einer Menge von 0,0005 bis 0,5 Gew.-% in geeignete Träger eingesetzt. Sie lassen sich problemlos in verschiedene pharmazeutische und kosmetische Träger einarbeiten.

Als kosmetische Träger eignen sich alle für die Aufbringung auf die Haare oder die Haut geeigneten Zubereitungen. Für die Hautbehandlung eignen sich insbesondere wäßrige oder alkoholische Lösungen, tensidhaltige Lotionen, Öle, Salben, Emulsionen, Cremes, Gele und Stiftpräparate. Für die Haarbehandlung eignen sich besonders Haarwässers, Haarshampoos, Haarkuren, Haarspülungen und Haarspray. Wegen der besonderen kosmetischen Problem, die durch fettendes Haar verursacht werden, stellen die haarkosmetischen Zubereitungen besonders bevorzugte Ausführungsformen der Erfindung dar.

Die wichtigsten Komponenten üblicher kosmetischer Träger sind

- Ölkomponenten z. B. Paraffinöl, Pflanzenöle, Fettsäureester, Squalan, Fettalkohole, 2-Octyldodecanol,
- Fette und Wachse, z. B. Walrat, Bienenwachs, Montanwachs, Paraffin, Cetyl-stearylalkohol,
- Emulgatoren, z. B. Fettsäurepartialglyceride, Fettsäure-Sorbitan-partialester und deren Ethoxylate, Seifen, Fettalkoholsulfate, Fettalkoholpolyglycolether, Alkylphosphate,
- Waschrohstoffe, insbesondere Aniontenside, z. B. Fettalkoholpolyglycolethersulfate, Fettalkoholsulfate, Alphaolefinsulfonate, Alkansulfonate, Sulfobernsteinsäureester, Acyltauride, Acylisethionate und Acylsarkosine,
  ampholytische Tenside, z.B. N-Alkylglycin, N-Alkylaminopropionsäure, N-Alkylaminobuttersäure mit 8 bis 18 C-Atomen in der Alkylgruppe, zwitterionische Tenside, z. B. N-Alkyl($C_8$-$C_{18}$)- N,N-dimethylammonio-glycinat oder N-Kokosacylaminopropyl-N,N-dimethylammonioglycinat und

nichtionogene Tenside, z. B. Fettalkoholpolyglycolether, Alkylphenolpolyglycoether, Fettsäurepolyglycolester, Aminoxid-Tenside, Fettsäurealkanolamide und deren Ethoxylate und kationische Tenside, z. B. Alkyl($C_{12}$-$C_{18}$)-trimethylammoniumchlorid, Lauryldimethylbenzylammoniumchlorid, Cetylpyridiniumchlorid, Distearyldimethylammoniumchlorid

- niedere Alkohole wie z. B. Ethanol, Isopropanol,
- mehrwertige Alkohole wie z. B. Ethylenglycol, Propylenglycol, Glycerin,
- Wasser und Hilfsstoffe wie z. B. Duftstoffe, Konservierungsmittel, Puffersubstanzen, Verdickungsmittel, Farbstoffe und Trübungsmittel.

Die folgenden Beispiele sollen den Erfindungsgegenstand näher erläutern:

## Beispiele

1. Herstellungsbeispiele

1.1 4- 4-(1,1,3-Trimethyl-2-cyclohexyl)-2-butoxy -benzoesäure-methylester

Die Mischung aus 11,7 g (77 mMol) 4-Hydroxy-benzoesäuremethylester, 200 ml N-Methylpyrrolidon und 13,8 g (77 mMol) 30%ige Natriummethylatlösung wurde nach dem Abdestillieren des Methanols mit 0,6 g Tetrabutylammoniumiodid und 20,0 g (92 mMol) 4-(1,1,3-Trimethyl-2-cyclohexyl)-2-butylchlorid versetzt und unter Rühren 8 Stunden auf 160 °C erwärmt. Nach Abkühlen auf Raumtemperatur wurde vom gebildeten Natriumchlorid abfiltriert, die Lösung unter vermindertem Druck zur Trockne gedampft, der Eindampfrückstand in Methylenchlorid aufgenommen, mit Wasser gewaschen, mit Natriumsulfat getrocknet, nach dem Eindampfen an Kieselgel (Merck) chromatographiert (Elutionsmittel: Methylenchlorid/Toluol = 7 : 3). Es wurden 13,9 g (54 % d. Th.) 4-(4-(1,1,3-Trimethyl-2-cyclohexyl)-2-butoxy)-benzoesäuremethylester als farbloses Öl vom Brechungsindex n20 : 1,5197 erhalten.

1.2 4-Cyclohexylmethoxy-zimtsäure-methylester

(Schmelzpunkt: 104 °C)

wurde nach dem in 1.1 beschriebenen Verfahren ausgehend von 4-Hydroxyzimtsäuremethylester und Chlormethylcyclohexan hergestellt.

1.3 4-(3-Cyclohexylpropyloxy)-benzoesäure-methylester

(Schmelzpunkt: 53 bis 54 °C)

wurde nach dem in 1.1 beschriebenen Verfahren ausgehend von 4-Hydroxybenzoesäuremethylester und 3-Cyclohexyl-1-chlorpropan hergestellt.

1.4 4-(4-(1,1,3-Trimethyl-2-cyclohexyl)-2-butoxy)-benzoesäure

Die Mischung aus 9 g (27 mMol) 4-(4-(1,1,3-Trimethyl-2-cyclohexyl)-2-butoxy)-benzoesäure-methylester (1.1), 20 ml 2-Propanol, 15 ml Wasser und 1,3 g Natriumhydroxid wurde 2 Stunden zum Sieden erhitzt, mit konz. Salzsäure angesäuert (pH 1). Nach dreimaligem Ausschütteln mit tert. Butylmethylether, Waschen der organischen Phase mit Wasser, Trocknen mit Natriumsulfat und Eindampfen wurde der Rückstand aus n-Hexan umkristallisiert. Es wurden 6 g (69 % d. Th.) farblose 4-(4-(1,1,3-Trimethyl-2-cyclohexyl)-2-butoxy)-benzoesäure vom Schmelzpunkt 114 bis 118 °C erhalten.

1.5 4-(Cyclohexylmethoxy)-zimtsäure

(Schmelzpunkt: 226 bis 228 °C)

wurde nach dem Verfahren gemäß 1.4 aus dem Methylester (1.2) hergestellt.

1.6 4-(3-Cyclohexylpropyloxy)-benzoesäure

(Schmelzpunkt: 182 bis 183 °C)

wurde nach dem Verfahren gemäß 1.4 aus dem Methylester (1.3) hergestellt.

1.7 4-Cyclohexylmethoxy-zimtalkohol

Zu der Lösung von 10 g (36,4 mMol) 4-Cyclohexylmethoxyzimtsäuremethylester (1.2) in 60 ml Toluol wurden unter Eiskühlung und Rühren 7,4 g (36,4 mMol) Vitride, 70%ig (Natrium-bis(2-methoxy-ethoxy)-aluminium-dihydrid) bei 5 bis 15 °C getropft. Anschließend wurde die Mischung 2 Stunden zum Sieden erhitzt, abgekühlt und unter Eiskühlung tropfenweise mit 10%iger Natronlauge versetzt (bis zum Ende der Wasserstoffentwicklung). Nach Zugabe von ca. 10 ml Wasser und einigen Tropfen 2-Propanol wurde die Toluol-Phase abgetrennt, die wäßrige Phase noch zweimal mit Toluol ausgewaschen, die vereinigten Toluol-Phasen mit Wasser neutral gewaschen und nach Trocknen mit Natriumsulfat eingedampft. Der Rückstand wurde an Kieselgel (Merck) chromatographiert (Elutionsmittel: Methylenchlorid/Methanol = 99 : 1). Es wurden 6,6 g (73 % d. Th.) 4-Cyclohexylmethoxyzimtalkohol als wachsartige Substanz erhalten.

1.8 4-Farnesyloxy-benzoesäure-methylester

Die Mischung aus 20,0 g (131 mMol) 4-Hydroxy-benzoesäuremethylester, 200 ml N-Methylpyrrolidon und 25,9 g (144 mMol) 30%ige Natriumethylatlösung wurde nach dem Abdestillieren des Methanols mit

1,1 g Tetrabutylammoniumiodid und 38,0 g (158 mMol) Farnesylchlorid versetzt und unter Rühren 7 Stunden auf 160 °C erwärmt. Nach Abkühlen auf Raumtemperatur wurde vom gebildeten Natriumchlorid abfiltriert, die Lösung unter vermindertem Druck zur Trockne gedampft, der Eindampfrückstand in Methylenchlorid aufgenommen, mit Wasser gewaschen, mit Natriumsulfat getrocknet, nach dem Eindampfen unter vermindertem Druck destilliert und die Fraktion mit dem Siedepunkt von 190 bis 202 °C/0,015 mbar an Kieselgel (Merck) chromatographiert (Elutionsmittel: Methylenchlorid/Toluol = 7 : 3). Es wurden 19,2 g (41 % d. Th.) 4-Farnesyloxy-benzoesäure-methylester als farbloses Öl vom Brechungsindex n20 : 1,5293 erhalten.

1.9 4-Geranyloxy-benzoesäure-methylester
($n^{20}$ : 1,5300; Schmelzpunkt: 30 bis 33 °C)
wurde nach dem in 1.8 beschriebenen Verfahren ausgehend von Geranylchlorid hergestellt.

1.10 4-Phytyloxy-benzoesäure-methylester
($n^{20}$ : 1,4995)
wurde nach dem in 1.8 beschriebenen Verfahren ausgehend von Phytylchlorid hergestellt.

1.11 3-(4-Farnesyloxy-phenyl)-propionsäure-methylester
($n^{20}$ : 1,5162)
wurde nach dem in 1.8 beschriebenen Verfahren ausgehend von 3-(4-Hydroxyphenyl)-propionsäuremethylester und Farnesylchlorid hergestellt.

1.12 4-Farnesyloxy-zimtsäure-methylester
($n^{20}$ : 1,5571)
wurde nach dem in 1.8 beschriebenen Verfahren ausgehend von 4-Hydroxyzimtsäuremethylester und Farnesylchlorid hergestellt.

1.13 3-(4-Farnesyloxy-phenyl)-propionsäure
Die Mischung aus 12 g (31,2 mMol) 3-(4-Farnesyloxy-phenyl)-propionsäure-methylester, 25 ml 2-Propanol, 25 ml Wasser und 1,4 g Natriumhydroxid wurde 5 Stunden zum Sieden erhitzt, mit konz. Salzsäure angesäuert (pH 1). Nach dreimaligem Ausschütteln mit tert. Butylmethylether, Waschen der organischen Phasen mit Wasser, Trocknen mit Natriumsulfat und Eindampfen wurde der Rückstand an Kieselgel (Merck) chromatographiert (Elutionsmittel: Methylenchlorid/Methanol = 96 : 4). Nach dem Eindampfen und Trocknen im Ölpumpenvakuum wurden 8,3 g (75 % d. Th.) 3-(4-Farnesyloxy-phenyl)-propionsäure als hellgelbes, viskoses Öl vom Brechungsindex n20 = 1,5041 erhalten.

1.14 4-Farnesyloxy-zimtsäure
(Schmelzpunkt: 70 bis 73 °C)
wurde nach dem Verfahren analog 1.13 ausgehend von 1.12 hergestellt.

1.15 4-Phytyloxy-benzoesäure
(zähe hochviskose Masse)
wurde nach dem Verfahren analog 1.13 ausgehend von 1.10 hergestellt.

1.16 4-Farnesyloxy-benzoesäure
(Schmelzpunkt: 63 bis 71 °C)
wurde nach dem Verfahren analog 1.13 ausgehend von 1.8 hergestellt.

1.17 4-Farnesyloxy-benzylalkohol
Zu der Lösung von 5 g (14 mMol) 4-Farnesyloxy-benzoesäuremethylester in 30 ml Toluol wurden unter Eiskühlung und Rühren 4 g (14 mMol) Vitride, 70%ig (Natrium-bis(2-methoxy-ethoxy)-aluminium-dihydrid) bei 5 bis 15 °C getropft. Anschließend wurde die Mischung 2 Stunden zum Sieden erhitzt, abgekühlt und unter Eiskühlung tropfenweise mit 10%iger Natronlauge versetzt (bis zum Ende der Wasserstoffentwicklung). Nach Zugabe von ca. 10 ml Wasser und einigen Tropfen 2-Propanol wurde die Toluol-Phase abgetrennt, die wäßrige Phase noch zweimal mit Toluol ausgewaschen und nach Trocknen mit Natriumsulfat eingedampft. Der Rückstand wurde an Kieselgel (Merck) chromatographiert (Elutionsmittel: Methylenchlorid/Methanol = 99 : 1). Es wurden 3,6 g (78 % d. Th.) 4-Farnesyloxy-benzylalkohol vom Brechungsindex n20 = 1,5305.

## 2. Prüfung und Bewertung der antiseborrhoischen Wirkung

### Grundlage

Der Test geht von der Beobachtung aus, daß männliche Ratten ein bräunliches Hautfett absondern, so daß die mehr oder weniger starke Fettigkeit der Haut visuell gut als Hautbräunung beurteilt werden kann. Daß es sich bei der Bräunung um Hautoberflächenfett handelt, ist daran zu erkennen, daß junge weibliche Ratten sowie männliche Ratten nach dem Waschen mit Tensidlösungen oder mit Lipidlösungsmitteln oder

auch männliche Ratten, die systematisch mit Östrogen behandelt wurden, nur die normale helle, rosafarbene Haut nach dem Scheren aufweisen. Parallel dazu sind aus den abgeschnittenen Haaren nur noch sehr geringe Lipidmengen zu extrahieren (vgl. hierzu auch J. Soc. Cosmet. Chem. 34, 127 - 135 (1983)).

Durchführung

Als Versuchstiere dienten männliche Wistar-Ratten mit einem Körpergewicht von 220 bis 230 g zu Versuchsbeginn.

Zur Beurteilung der Wirksamkeit wurden die Prüfsubstanzen in den in Tabelle 1 angegebenen Konzentrationen in Ethanol/Aceton (1 : 1) jeweils 6 Ratten halbseitig auf das Rückenfell gepinselt. Die andere Seite wurde nur mit dem Lösungsmittel behandelt.

Während der Versuchsdauer von 14 Tagen wurde an insgesamt 9 Tagen einmal appliziert. Zur weiteren Kontrolle diente eine Gruppe von 6 Ratten, die beidseitig nur mit dem Lösungsmittel behandelt wurden. Am Ende des Versuchs wurden die Tiere am Rücken und an den Flanken geschoren und von einem Beurteilerpanel (6 Personen) unabhängig unter Doppelblindbedingungen visuell abgemustert. Dabei wurde der Bräunungsgrad auf dem Rücken der Ratten als Maß für den Hautfettbelag visuell beurteilt.

Bewertung

Als 1. Kriterium wurde der Unterschied zwischen rechter und linker Seite gewertet, wobei pro Beurteiler und Tier jeweils 1 Punkt zu vergeben war, und zwar in der Weise, daß die

| | |
|---|---|
| dunklere Seite | mit 1 Punkt |
| hellere Seite | mit 0 Punkten und |
| bei Gleichheit beide Seiten | mit 0,5 Punkten |

benotet wurden.

Signifikante Differenzen zwischen nur mit dem Lösungsmittel behandelter und mit Prüflösung behandelter Seite nach dieser Bewertungsmethode zeigen die lokale Wirksamkeit einer Substanz an.

Als 2. Kriterium wurden außerdem noch die Intensitätsunterschiede der Brauntöne nach folgender Skala bewertet:

| | |
|---|---|
| 3 Punkte | stark braun |
| 2 Punkte | mittel braun |
| 1 Punkt | schwach braun |
| 0 Punkte | keine Braunfärbung. |

Nach dieser Bewertungsmethode werden die Punktsummendifferenzen zwischen den nur mit dem Lösungsmittel behandelten Kontrolltieren und jeweils den behandelten und unbehandelten Seiten ($\Delta P$) der Versuchstiere gebildet, wobei wiederum signifikante Differenzen zwischen Kontrolltieren und der behandelten Seite der Versuchstiere die Wirkung einer Substanz deutlich machen.

Prozentuale Sebumreduktion

Die Sebumreduktion errechnet sich aus der Punktedifferenz in der Weise, daß man den Quotienten aus der Punktedifferenz $\Delta P$ und der Punktezahl für die Kontrollgruppe $P_k$ bildet und den erhaltenen Wert in % angibt.

$$\text{Sebumreduktion} = \frac{\Delta P}{P_k} \cdot 100 \quad (\%)$$

Die Ergebnisse für die Substanzen 1.1 bis 1.17 sind der Tabelle 1 zu entnehmen:

Tabelle 1

| Produkt gemäß Beispiel | Konzentration Gew.-% | Prozentuale Sebumreduktion |
|---|---|---|
| 1.1 | 0,01 | 25 |
| 1.1 | 0,05 | 99 |
| 1.2 | 0,5 | 29 |
| 1.3 | 0,02 | 0 |
| 1.3 | 0,05 | 68 |
| 1.3 | 0,5 | 99 |
| 1.4 | 0,001 | 73 |
| 1.4 | 0,005 | 88 |
| 1.4 | 0,01 | 93 |
| 1.4 | 0,05 | 100 |
| 1.5 | 0,5 | 15 |
| 1.6 | 0,005 | 0 |
| 1.6 | 0,01 | 52 |
| 1.6 | 0,02 | 84 |
| 1.7 | 0,1 | 53 |
| 1.7 | 0,5 | 66 |
| 1.8 | 0,01 | 38 |
| 1.8 | 0,05 | 86 |
| 1.9 | 0,1 | 40 |
| 1.11 | 0,05 | 86 |
| 1.12 | 0,05 | 85 |
| 1.13 | 0,05 | 68 |
| 1.14 | 0,05 | 73 |
| 1.15 | 0,05 | 35 |
| 1.16 | 0,01 | 69 |
| 1.17 | 0,001 | 14 |
| 1.17 | 0,002 | 18 |
| 1.17 | 0,005 | 72 |
| 1.17 | 0,01 | 87 |
| 1.17 | 0,05 | 86 |

## 3. Erfindungsgemäße Zubereitungen

### 3.1 Shampoo für fettendes Haar

| | | | |
|---|---|---|---|
| Texapon(R) N 25 | (1) | 40 | Gew.-% |
| Comperlan(R) KD | (2) | 3 | |
| Produkt nach Beispiel 1.4 | | 0,1 | |
| Bronidox(R) L | (3) | 0,2 | |
| Wasser | | ad 100 | |

### 3.2 Schnellhaarkur-Emulsion

| | | | |
|---|---|---|---|
| Cetylalkohol | | 3,0 | Gew.-% |
| Dehyquart(R) A | (4) | 2,0 | |
| Produkt nach Beispiel 1.6 | | 0,02 | |
| Citronensäure | | 1,0 | |
| Wasser | | ad 100 | |

### 3.3 Schnellhaarkur, klar

| | | | |
|---|---|---|---|
| Cetiol(R) HE | (5) | 20,0 | Gew.-% |
| Cetylpyridiniumchlorid | | 5,0 | |
| Glycerin | | 5,0 | |
| Produkt nach Beispiel 1.8 | | 0,05 | |
| Isopropanol | | ad 100 | |

### 3.4 Haarwasser

| | | | |
|---|---|---|---|
| Cetiol(R) HE | (5) | 2,0 | Gew.-% |
| Birkenextrakt | | 1,0 | |
| Produkt nach Beispiel 1.17 | | 0,005 | |
| Isopropanol | | 30,0 | |
| Wasser | | ad 100 | |

3.5 Hautemulsion O/W

| | | | |
|---|---|---|---|
| Cutina(R)MD | (6) | 7,0 | Gew.-% |
| Eumulgin(R)B1 | (7) | 3,0 | |
| Cetiol(R)SN | (8) | 10,0 | |
| Myritol(R)318 | (9) | 10,0 | |
| Produkt nach Beispiel 1.4 | | 0,01 | |
| Wasser | | ad 100 | |

3.6 Hautcreme O/W

| | | | |
|---|---|---|---|
| Cutina(R)MD | (6) | 17 | Gew.-% |
| Eumulgin(R)B1 | (7) | 3 | |
| Eutanol(R)G | (10) | 11 | |
| Myritol(R)318 | (9) | 6 | |
| Karottenöl CLR | | 3 | |
| Produkt nach Beispiel 1.1 | | 0,02 | |
| Wasser | | ad 100 | |

Die in den Rezepturbeispielen verwendeten Handelsnamen haben folgende Bedeutung:

| | |
|---|---|
| (1) Texapon®N 25: | 28%ige wäßrige Lösung von Alkyl-($C_{12}$-$C_{14}$)poly(2 EO)glycolethersulfat-Na-Salz (Henkel KGaA) |
| (2) Comperlan®KD: | Kokosfettsäurediethanolamid (Henkel KGaA) |
| (3) Bronidox®L: | 5-Brom-5-nitro-1,3-dioxan (10%ige Lösung in 1,2-Propylenglycol) (Henkel KGaA) |
| (4) Dehyquart®A: | Cetyltrimethylammoniumchlorid (25%ige Lösung in Wasser) (Henkel KGaA) |
| (5) Cetiol®HE: | Polyol-Fettsäureester (CTFA-Bezeichnung: PEG-7-Glyceryl-Cocoate) (Henkel KGaA) |
| (6) Cutina®MD: | Palmitin/stearinsäure-mono/diglycerid (Henkel KGaA) |
| (7) Eumulgin®B1: | Cetyl/stearylalkohol + 12 Mol Ethylenoxid (Henkel KGaA) |
| (8) Cetiol®SN: | Cetyl/stearyl-isononanoat (Henkel KGaA) |
| (9) Myritol®318: | Capryl/caprinsäure-triglycerid (Henkel KGaA) |
| (10) Eutanol®G: | 2-Octyldodecanol (Henkel KGaA) |

## Patentansprüche

**1.** Verwendung von Alkyl- und Alkenyl-Arylether-Derivaten der Formel (I)

(I) 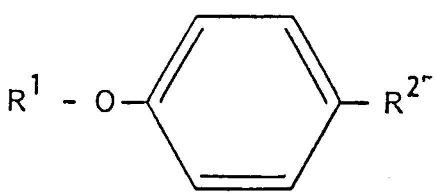

in der $R^1$ eine gesättigte, cycloaliphatische Alkylgruppe mit 6 bis 20 C-Atomen oder eine einfach oder mehrfach ungesättigte, lineare, verzweigte oder cycloaliphatische Alkylgruppe mit 6 bis 20 C-Atomen und $R^2$ eine Hydroxyalkylgruppe mit 1 bis 4 C-Atomen, eine Hydroxyalkenylgruppe mit 3 oder 4 C-Atomen, eine Gruppe $-COOR^3$, $-CH_2-COOR^3$, $-CH_2-CH_2-COOR^3$ oder $-CH=CH-COOR^3$ ist, worin $R^3$ eine Alkylgruppe mit 1 bis 4 C-Atomen, eine Hydroxyalkylgruppe mit 2 bis 4 C-Atomen, eine Alkoxyalkylgruppe mit 1 bis 4 C-Atomen in der Alkoxy-und 2 bis 4 C-Atomen in der Alkylgruppe oder

Wasserstoff oder ein salzbildendes Kation ist, als antisborrhoische Wirkstoffe zur Herstellung von topischen, pharmazeutischen und kosmetischen Mitteln mit sebosuppressiver Wirkung.

**2.** Verwendung nach Anspruch 1, dadurch gekennzeichnet, daß in den Alkyl- und Alkenyl-Aryletherderivaten der Formel (I) $R^1$ eine Cyclohexylalkyl- oder eine Mono-, Di- oder Trimethyl-cyclohexyl-alkylgruppe mit insgesamt 6 bis 20 C-Atomen und $R^3$ eine Gruppe $-CH_2OH$, $-CH=CH-CH_2OH$, $-COOR^3$, $-CH_2-CH_2-COOR^3$ oder $-CH=CH-COOR^3$ ist, worin $R^3$ Wasserstoff, ein salzbildendes Kation oder eine Alkylgruppe mit 1 bis 4 C-Atomen ist.

**3.** Verwendung nach Anspruch 1, dadurch gekennzeichnet, daß in den Alkyl- und Alkenyletherderivaten der Formel (I) $R^1$ eine von ungesättigten Terpenalkohlen abgeleitete Gruppe, bevorzugt eine Geranyl-, Neryl-, Nerolidyl-, Farnesyl-, 6,10-Dimethyl-5-9-undecadienyl-, beta-Citronellyl-, Phytyl-oder Isophytylgruppe ist.

**4.** Sebosuppressive Zubereitungen zur topischen Anwendung auf dem Haar und auf der Haut, bestehend aus antiseborrhoischen Wirkstoffen in einem dermatologisch verträglichen kosmetischen oder pharmazeutischen Träger, dadurch gekennzeichnet, daß als antiseborrhoischer Wirkstoff ein Alkyl- und/oder Alkenyl-Arylether der Formel (I)

(I) 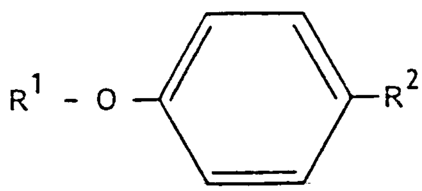

in der $R^1$ eine gesättigte, cycloaliphatische Alkylgruppe mit 6 bis 20 C-Atomen oder eine einfach oder mehrfach ungesättigte, lineare, verzweigte oder cycloaliphatische Alkylgruppe mit 6 bis 20 C-Atomen ist und $R^2$ eine Hydroxyalkylgruppe mit 1 bis 4 C-Atomen, eine Hydroxyalkenylgruppe mit 3 oder 4 C-Atomen, ein Gruppe $-COOR^3$, $-CH_2-COOR^3$, $-CH_2-CH_2-COOR^3$ oder $-CH=CH-COOR^3$ ist, worin $R^3$ eine Alkylgruppe mit 1 bis 4 C-Atomen, eine Hydroxyalkylgruppe mit 2 bis 4 C-Atomen, eine Alkoxyalkylgruppe mit 1 bis 4 C-Atomen in der Alkoxy- und 2 bis 4 C-Atomen in der Alkylgruppe, Wasserstoff oder ein salzbildendes Kation ist, in einer Menge von 0,0005 bis 0,5 Gew.-% bezogen auf die gesamte Zubereitung, enthalten ist.

## Claims

**1.** The use of alkyl and alkenyl aryl ether derivatives corresponding to formula (I)

(I) 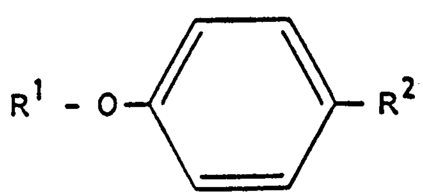

in which $R^1$ is a saturated, cycloaliphatic $C_{6-20}$ alkyl group or a mono- or polyunsaturated, linear, branched or cycloaliphatic $C_{6-20}$ alkyl group and $R^2$ is a $C_{1-4}$ hydroxyalkyl group, a $C_3$ or $C_4$ hydroxyalkenyl group, a group $-COOR^3$, $-CH_2-COOR^3$, $-CH_2-CH_2-COOR^3$ or $-CH=CH-COOR^3$, where $R^3$ is a $c_{1-4}$ alkyl group, a $C_{2-4}$ hydroxyalkyl group, an alkoxyalkyl group with 1 to 4 C atoms in the alkoxy group and 2 to 4 C atoms in the alkyl group or hydrogen or a salt-forming cation, as antiseborrhoeic agents for the preparation of topical, pharmaceutical and cosmetic sebosuppressive preparations.

**2.** The use claimed in claim 1, characterized in that, in the alkyl and alkenyl aryl ether derivatives of formula (I), $R^1$ is a cyclohexylalkyl group or a mono-, di- or trimethyl cyclohexyl alkyl group containing

in all 6 to 20 carbon atoms and $R^3$ is a group $-CH_2OH$, $-CH=CH-CH_2OH$, $-COOR^3$, $-CH_2-CH_2-COOR^3$ or $-CH=CH-COOR^3$, where $R^3$ is hydrogen, a salt-forming cation or a $C_{1-4}$ alkyl group.

**3.** The use claimed in claim 1, characterized in that, in the alkyl and alkenyl ether derivatives of formula (I), $R^1$ is a group derived from unsaturated terpene alcohols, preferably a geranyl, neryl, nerolidyl, farnesyl, 6,10-dimethyl-5,9-undecadienyl, $\beta$-citronellyl, phytyl or isophytyl group.

**4.** Sebosuppressive preparations for topical application to the hair and to the skin consisting of antiseborrhoeic agents in a dermatologically safe cosmetic or pharmaceutical vehicle, characterized in that they contain an alkyl and/or alkenyl aryl ether corresponding to formula (I)

(I) 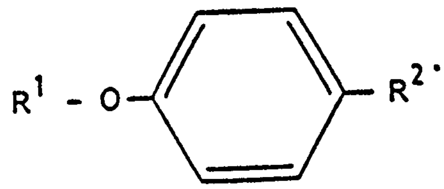

in which $R^1$ is a saturated, cycloaliphatic $C_{6-20}$ alkyl group or a mono- or polyunsaturated, linear, branched or cycloaliphatic $c_{6-20}$ alkyl group and $R^2$ is a $C_{1-4}$ hydroxyalkyl group, a $C_3$ or $C_4$ hydroxyalkenyl group, a group $-COOR^3$, $-CH_2-COOR^3$, $-CH_2-CH_2-COOR^3$ or $-CH=CH-COOR^3$, where $R^3$ is a $C_{1-4}$ alkyl group, a $C_{2-4}$ hydroxyalkyl group, an alkoxyalkyl group with 1 to 4 C atoms in the alkoxy group and 2 to 4 C atoms in the alkyl group or hydrogen or a salt-forming cation,
as antiseborrhoeic agent in a quantity of 0.0005 to 0.5% by weight, based on the preparation as a whole.

## Revendications

**1.** Utilisation de dérivés de type alkyle-et alcénylarylique de formule (I)

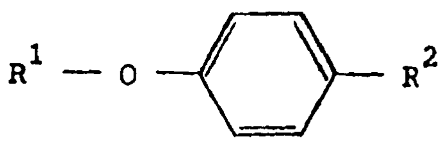

dans laquelle $R^1$ est un groupe alkyle cycloaliphatique saturé ayant de 6 à 20 atomes de carbone, un groupe alkyle linéaire, ramifié ou cycloaliphatique une ou plusieurs fois insaturé, ayant de 6 à 20 atomes de carbone, et $R^2$ est un groupe hydroxyalkyle ayant de 1 à 4 atomes de carbone, un groupe hydroxyalcényle ayant 3 ou 4 atomes de carbone, un groupe $-COOR^3$, $-CH_2-COOR^3$, $-CH_2-CH_2-COOR^3$ ou $-CH=CH-COOR^3$, $R^3$ étant un groupe alkyle ayant de 1 à 4 atomes de carbone, un groupe hydroxyalkyle ayant de 2 à 4 atomes de carbone, un groupe alcoxy-alkyle ayant de 1 à 4 atomes de carbone dans le fragment alcoxyl et de 2 à 4 atomes de carbone dans le fragment alkyle, ou un atome d'hydrogène ou un cation solifiant, en tant que substances actives anti-séborrhéiques pour la fabrication de produits pharmaceutiques et cosmétiques topiques à action sébosuppressive.

**2.** Utilisation selon la revendication 1, cractérisés en ce que, dans les dérivés de type éther alkyle- et alcénylarylique de formule (I), $R^1$ est un groupe cyclohexylalkylique ou un groupe mono-, di-ou triméthylcyclohexylalxylique ayant au total de 6 à 20 atomes de carbone, et $R^3$ est un groupe $-CH_2OH$, $-CH\ =CH-CH_2OH$, $-COOR^3$, $-CH_2-CH_2-COOR^3$ ou $-CH=CH-COOR^3$, $R^3$ étant un atome d'hydrogène, un cation salifiant ou un groupe alkyle ayant de 1 à 4 atomes de carbone.

**3.** Utilisation selon la revendication 1, caractérisée en ce que, dans les dérivés de type éther alkyle- et alcényliques de formule (I), $R^1$ est un groupe insaturé dérivé d'un alcool terpénique, de préférence un groupe géranyle, néryle, nérolidyle, farnésyle, 6, 10-diméthyl-5,9-undécadiényle, bêtacitronellyle,phytyle ou isophytyle.

**4.** Compositions sébosuppressives pour l'application locale sur les cheveux ou sur la peau, constituées de substances actives anti séborrhériques dans un véhicule pharmaceutique ou cosmétique dermatologiquement acceptable, caractérisée en ce que, en tant que substance active anti-séborrhérique, est contenu un ester alkyle- et/ou alcényl-arylique de formule (I)

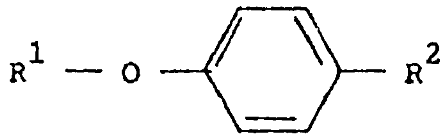

(I)

dans laquelle $R^1$ est un groupe alkyle cycloalphatique saturé ayant de 6 à 20 atomes de carbone ou un groupe alkyle linéaire, ramifié ou cycloaliphatique saturé ayant de 6 à 20 atomes de carbone et $R^2$ est un groupe hydroxyalkylique ayant de 1 à 4 atomes de carbone, un groupe hydroxy-alcényle ayant 3 ou 4 atomes de carbone, un groupe $-COOR^3$, $-CH_2-COOR^3$, $-CH_2-CH_2-COOR^3$ ou $-CH=CH-COOR^3$, $R^3$ étant un groupe alkyle ayant de 1 à 4 atomes de carbone, un groupe hydroxyalkyle ayant de 2 à 4 atomes de carbone, un groupe alcoxyalkyle ayant de 1 à 4 atomes de carbone dans le fragment alcoxyl et de 2 à 4 atomes de carbone dans le fragment alkyle, un atome d'hydrogène ou un cation salifiant, en une quantité de 0,0005 à 0,5 % en poids, par rapport à la composition totale.